**Europäisches Patentamt**

**European Patent Office**

**Office européen des brevets**

(19)

(11) Publication number : **0 351 734 B1**

## (12) EUROPEAN PATENT SPECIFICATION

(45) Date of publication of patent specification :
09.09.92 Bulletin 92/37

(51) Int. Cl.⁵ : **A61M 25/10**

(21) Application number : **89112961.1**

(22) Date of filing : **14.07.89**

(54) Balloon catheter.

(30) Priority : **18.07.88 JP 179650/88**

(43) Date of publication of application :
**24.01.90 Bulletin 90/04**

(45) Publication of the grant of the patent :
**09.09.92 Bulletin 92/37**

(84) Designated Contracting States :
**BE DE FR GB IT NL SE**

(56) References cited :
**WO-A-82/03557**
**US-A- 4 342 316**

(73) Proprietor : **TERUMO KABUSHIKI KAISHA**
**No. 44-1, Hatagaya 2-chome, Shibuya-ku**
**Tokyo 151 (JP)**

(72) Inventor : **Harada, Kinya**
**c/o Terumo Kabushiki Kaisha 2827,**
**Mannoharashinden**
**Fujinomiya-shi Shizuoka-ken (JP)**

(74) Representative : **Casalonga, Axel et al**
**BUREAU D.A. CASALONGA - JOSSE**
**Morassistrasse 8**
**W-8000 München 5 (DE)**

## Description

This invention relates to a balloon catheter adapted for use by inserting it in a body cavity and indwelling thereat, and more particularly, to such a balloon catheter adapted for use in measuring the cardiac output.

For cardiac function examination, a balloon catheter is often used to measure the cardiac output. Such measurement is carried out by introducing the distal end of the catheter into the vein through the femoral vein or the like by means of an insertion aid such as an introducer, causing the balloon to inflate, allowing the distal end to be transferred to the desired site along with the flow of blood, thereafter causing the balloon to contract, and indwelling the catheter at the site. The catheter is then ready for measurement.

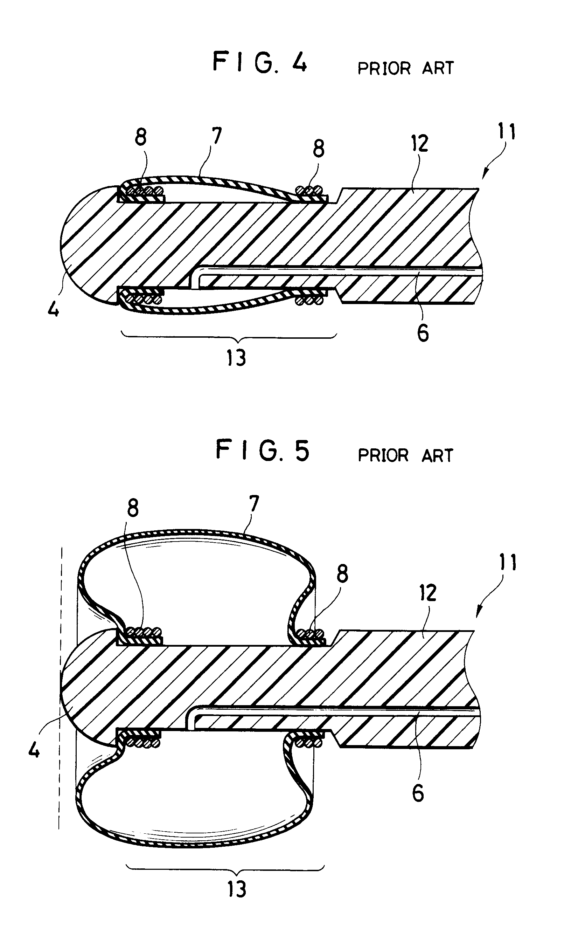

FIG. 4 illustrates one typical example of the prior art balloon catheter. The prior art balloon catheter generally designated at 11 includes an elongated member 12 having a head 4 defining a distal end and an attachment portion 13 formed adjacent to the head. A generally cylindrical balloon 7 formed of elastomeric material is secured to the attachment portion 13 by binding with thread 8. When balloon 7 is expanded as shown in FIG. 5, it is essential that the balloon fully surround the head 4 defining the distal end, that is, an extreme end of the expanded balloon axially extend beyond the distal end of head 4. If the head 4 is not surrounded by the expanded balloon 7, there is a risk that the head 4 which is more rigid than the balloon comes in contact with inner walls of blood vessels and the heart causing damages thereto.

Nevertheless, many prior art balloon catheters fail to meet this requirement. Since the attachment portion 13 of the prior art balloon catheter 11 is usually in the form of a straight cylinder having a fixed diameter, the balloon 7, when expanded, does not axially extend beyond the distal end of head 4 as indicated by a phantom line in FIG. 5, thus failing to fully surround head 4.

US-A-4.342.316 discloses a balloon catheter with a tapered end portion which has only the aim of facilitating insertion in the patient urethra during placement of the catheter.
WO-A-8.203.557 discloses a balloon catheter, the balloon of which protrudes in an expanded state axially beyond the distal end of the cylindrical elongated member of the catheter. The balloon has a differential thickness in order to obtain a specific shape of the inflated balloon, thus avoiding in a specific way, initiation of the bladder by the hard and pointed tip of the catheter.

An object of the present invention is to provide a novel and improved balloon catheter free of the risk of damaging the tissue of organs and vessels where the catheter is inserted.

The balloon catheter of the invention as claimed comprises
a generally cylindrical elongated member having an axis, a distal end and an attachment portion adjacent the distal end, an expandable/contractable balloon attached to the attachment portion of said elongated member, and
a lumen extending through the elongated member in communication with the interior of the balloon. The attachment portion is tapered toward the distal end having a smaller diameter at a location nearer to the distal end. According to the invention, the balloon has a substantially equal diameter throughout its axial length in a contracted state. Due to the combination of the tapered shape of the attachment portion with the specific shape of the balloon, the balloon axially protrudes beyond the distal end of said elongated member in an expanded state.

Preferably, the attachment portion is tapered at an angle of 0.85° to 1.45° relative to the elongated member axis.

The above and other objects, features, and advantages of the present invention will be better understood from the following description taken in conjunction with the accompanying drawings, of same examples of the invention in which:
FIG. 1 is an axial cross section of a balloon catheter according to one embodiment of the present invention, showing the balloon in a contracted condition;
FIG. 2 is an axial cross section of the balloon catheter of FIG. 1, showing the balloon in an expanded condition;
FIG. 3 is an axial cross section of a balloon catheter according to another embodiment of the present invention, showing the balloon in a contracted condition;
FIG. 4 is an axial cross section of a prior art balloon catheter, showing the balloon in a contracted condition; and
FIG. 5 is an axial cross section of the balloon catheter of FIG. 4, showing the balloon in an expanded condition.

Like parts are designated by the same reference numerals throughout the figures.

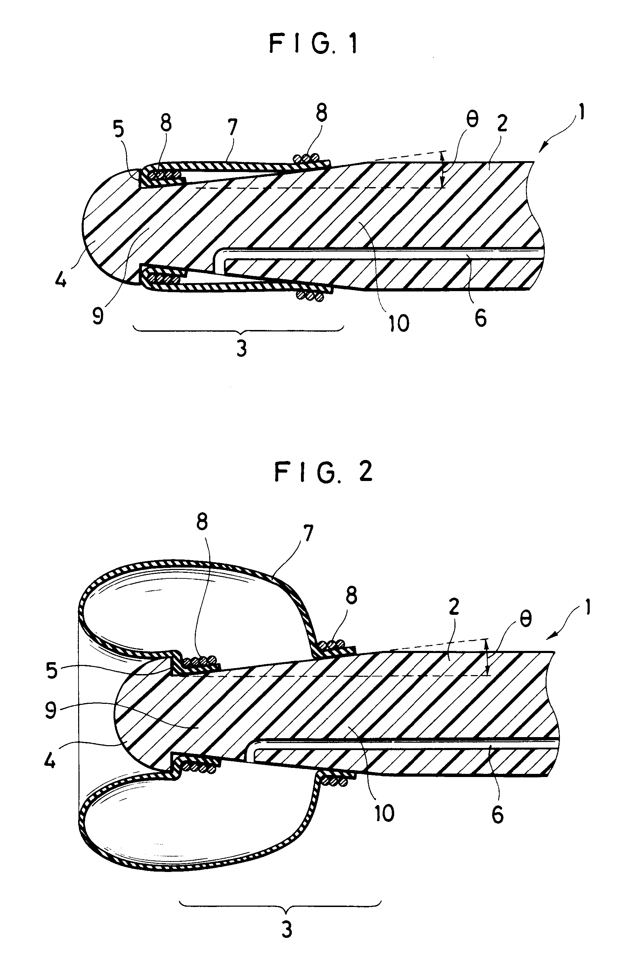

FIGS. 1 and 2 illustrate in an axial cross section a balloon catheter according to one embodiment of the present invention, with the balloon in contracted and expanded conditions.

The balloon catheter is generally designated at 1 as comprising a generally cylindrical elongated member or tube 2 including a head 4 at a distal or forward end and an attachment portion 3 adjacent thereto. An expandable/contractable balloon 7 is attached to the attachment portion 3 of the elongated member. A lumen 6 extends through the elongated member in communication with the interior of the balloon.

More particularly, the elongated member 2 includes the head 4 having a curved, preferably spherical surface and defining a distal end at the left in the figures. The attachment portion 3 having an axial length is defined in the elongated member 2 adjacent the head 4. The attachment portion 3 is tapered toward the distal end to form a step 5 between the attachment portion 3 and the head 4. Provision of the step 5 ensures that the outer diameter of the balloon 7 in a contracted condition becomes approximately equal to that of the elongated member 2, especially that of the head 4 as seen from FIG. 1. When the balloon catheter is inserted into the blood vessel with the aid of an introducing tool, there is no risk of balloon 7 being damaged by contacting interior walls of the introducing tool and associated check valve and other parts.

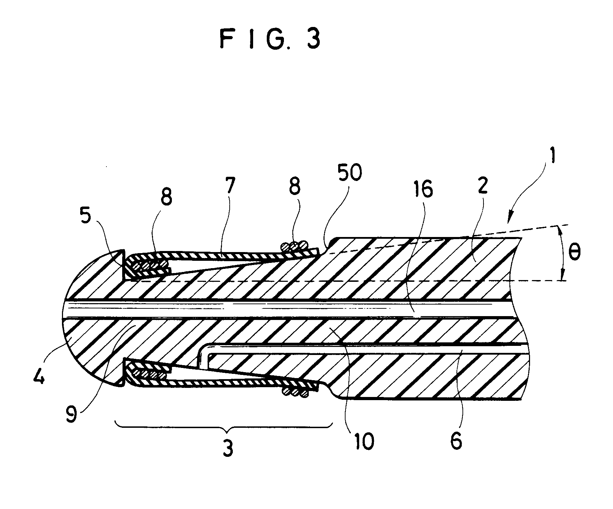

Another configuration of the attachment portion is shown in FIG. 3. In addition to the step 5 between the attachment portion 3 and the head 4, another step 50 is formed between the attachment portion 3 and the remaining portion (right side in FIG. 3) of the elongated member 2 remote from the head 4. Then the outer diameter of the balloon 7 in a contracted condition also becomes approximately equal to that of the elongated member 2 at locations remote from the head 4.

The elongated member 2 may be formed of any desired flexible or elastomeric material, for example, polyvinyl chloride, polyurethane, silicone rubber, polyethylene, nylon, and ethylene-vinyl acetate copolymers. The dimensions of the elongated member 2 is not critical as long as the elongated member is sufficiently slender to enter the blood vessel. Preferably, the elongated member 2 has a diameter of about 1.4 mm to 2.1 mm.

Since the catheter of the invention is generally used by inserting it in a body cavity and indwelling thereat, there is often a need for carrying out measurement or medical treatment to obtain a visual identification of the location of the catheter by photofluoroscopy. To this end, the catheter member is preferably made radiopaque. More particularly, a radiopaque or contrast agent is incorporated in the material of which the elongated member is made. Examples of the radiopaque agent include barium sulfate, bismuth oxide, and tungsten.

The elongated member 2 includes the axially extending lumen 6 forming a passage through which fluid is passed to and from the balloon 7 for expansion and contraction. The lumen 6 at one end opens in the peripheral wall within the attachment portion 3 so that the lumen 6 is in fluid communication with the interior of the balloon 7. The fluid used herein is not critical to the present invention, but is usually selected from physiologically safe fluids, for example, gases such as carbon dioxide and oxygen, and liquids such as physiological saline and glucose solution.

The elongated member 2 may include one or more other lumina in addition to the lumen 6. When the balloon catheter of the invention is used in measuring the cardiac output, the elongated member 2 may preferably be provided with a second lumen 16 axially extending therethrough and opening at the head 4 for measuring the blood pressure as shown in FIG. 3, a third lumen for introducing liquid indicator diluent, and a fourth lumen for receiving a thermistor and electrical leads therefor (the third and fourth lumina are not shown).

The balloon 7 having a generally cylindrical shape is attached to the outer peripheral wall of the attachment portion 3. The wall of balloon 7 extends over a substantial portion of the attachment portion 3 to cover the opening of lumen 6.

The balloon 7 may be formed by any desired elastic materials, for example, rubbery materials such as silicone rubber and latex rubber and plastic materials such as urethane, polyvinyl chloride and ethylene-vinyl acetate copolymers insofar as it is expandable and contractable. Before assembly, the balloon 7 may be a hollow cylindrical elastic member having a wall thickness of 0.08 to 0.20 mm, for example, although the exact wall thickness depends on a particular material.

The balloon 7 may be attached to the attachment portion 3 by preparing a cylindrical member having first and second margins and fitting the cylindrical member over the elongated member 2 from the forward end (4) until its leading edge extends a predetermined distance beyond the step 5. The first margin of the cylindrical member is bound to the forward area of the attachment portion 3 adjacent the head 4 by winding a length of thread 8 several turns and applying adhesive thereon. Then the remaining portion of the cylindrical member is turned inside out and extended over the elongated member until its other edge reaches the rear end of the attachment portion 3 remote from the head 4. The second margin of the cylindrical member is bound to the rear area of the attachment portion 3 remote from the head 4 by winding a length of thread 8 several turns and applying adhesive thereon. At this point there is obtained a structure as shown in FIG. 1 or 3.

The method of attaching the balloon 7 to the attachment portion 3 is not limited to the above-mentioned one. For example, the first and second attachment margins of the balloon-forming cylindrical member may be directly secured to the associated areas of the attachment portion 3 by adhesive bonding or fusion welding.

According to the feature of the present invention, the attachment portion 3 of the elongated member 2 is tapered toward the distal or forward end and has a smaller diameter at a location 9 nearer to the distal end. The attachment portion 3 has a smaller diameter portion 9 at the forward end and a larger diameter portion 10 at the rear end. The attachment portion 3 is tapered forward such that when the balloon 7 is inflated, the balloon 7 fully surrounds the head 4, that is, an extreme end of the balloon axially extends beyond the distal end of head 4 as shown in FIG. 2.

The reason will be briefly explained. The balloon 7 in a normal state has the general shape of a cylinder having a fixed diameter throughout its axial length. When such a balloon is engaged around the tapered attachment portion 3, the balloon is held to the smaller diameter portion 9 in a relatively loose fashion or at a lower degree of tension and to the larger diameter portion 10 in a relatively tight fashion or at a higher degree of tension. Then the balloon 7 in the contracted condition leaves some degree of looseness near the smaller diameter portion 9, tending to expand toward the distal end of the elongated member 2.

Preferably, the attachment portion 3 is tapered at an angle θ of 0.85° to 1.45° relative to the elongated member axis. (For brevity's sake, the taper angle θ is depicted as the angle between the tapered surface and a line parallel to the axis in FIGS. 1-3). A taper angle θ of less than 0.85° would be ineffective with some material and thickness of the balloon. A taper angle θ of more than 1.45° would sometimes prevent the balloon from uniformly inflating, obstructing smooth indwelling of the balloon. Better results were obtained when a taper angle of 1.1° was used in one example. A tapered attachment portion may be formed by machining a straight tubular member or molding a configured tubular member.

The balloon catheters of the present invention may be used as any types of balloon catheter including balloon catheters for cardiac output measurement, urinary balloon catheters, and PTCA (percutaneous transluminal coronary angioplasty) and PTA balloon catheters.

The balloon catheter of the present invention operates as follows. For example, the balloon catheter 1 may be set in place by inserting the catheter into the blood vessel, causing the balloon 7 to expand, and allowing the balloon 7 to be transferred with the blood flow until the distal or forward end of the catheter 1 reaches the desired site. More particularly, the balloon 7 is inflated by pumping an appropriate fluid into the lumen 6 through a connector at the proximal end of the catheter (not shown), thereby introducing the fluid into the balloon 7 through the opening of the lumen 6.

When inflated, the balloon 7 axially extends beyond the distal end of the head 4 and radially surrounds the head 4 as shown in FIG. 2. The desired inflation of the balloon 7 is achieved according to the above-described principle since the attachment portion 3 is tapered toward the distal end. The tapering of the attachment portion 3 at an angle θ of 0.85° to 1.45° ensures that the balloon 7 axially extends beyond the distal end of the head 4 when inflated, independent of a particular type of material, thickness and length of the balloon 7. Then the type of material and dimensions of the balloon 7 become less limited, with the advantage of more freedom of design.

With the balloon 7 in the expanded condition, it is the soft balloon 7 and not the relatively hard head 4 that comes in contact with inner walls of the vessel or heart when the leading edge of the balloon catheter is moved in the vessel or heart. There is little risk of damaging vessel or heart inner walls.

The balloon catheter has been described in which a balloon is affixed to a tapered attachment portion of an elongated member. The balloon when inflated axially extends beyond the distal end of the head and radially surrounds the head since the attachment portion is tapered toward the distal end. There is thus provided a balloon catheter which has little or no risk of damaging the tissue of a human body site where the catheter is introduced, including blood vessels and the heart.

Although some preferred embodiments have been described, many modifications and variations may be made thereto in the light of the above teachings. It is therefore to be understood that within the scope of the appended claims, the invention may be practiced otherwise than as specifically described.

## Claims

1. A balloon catheter comprising

a generally cylindrical elongated member (2) having an axis, a distal end and an attachment portion (3) adjacent the distal end, an expandable/contractable balloon (7) attached to the attachment portion of said elongated member, and

a lumen (6) extending through the elongated member (2) in communication with the interior of the balloon (7), the attachment portion (3) being tapered toward the distal end having a smaller diameter at a location nearer to the distal end, characterized in that the balloon (7) has a substantially equal diameter throughout its axial length in a contracted state;

whereby the balloon (7) axially protrudes beyond the distal end of said elongated member (2) in an expanded state.

2. The balloon catheter of claim 1 which further comprises a second lumen (16) axially extending through the elongated member (2) to the distal end.

3. The ballon catheter of claim 1 wherein the attachment portion is tapered at an angle of 0.85° to 1.45° relative to the elongated member axis.

4. The balloon catheter of any preceeding claim wherein the forward end portion of the balloon material is attached to the forward area of the attachment portion (3), the remaining portion of the balloon material is turned inside out and extended over the elongated member toward the rear end of the attachment portion, and the balloon material is applied against and attached to the rear end of the attachment portion (3).

## Patentansprüche

1. Ballon-Katheter, umfassend

ein im wesentlichen zylindrisches, langgestrecktes Element (2) mit einer Achse, einem distalen Ende und einem am distalen Ende befindlichen Anschlußabschnitt (3), wobei am Anschlußabschnitt des langgestreckten Elements ein aufweitbarer/zusammenziehbarer Ballon (7) angebracht ist, und

ein Lumen (6), das durch das langgestreckte Element (2) verläuft und mit dem Inneren des Ballons (7) in Verbindung steht, wobei sich der Anschlußabschnitt (3) zum distalen Ende hin verjüngt und an einer näher am distalen Ende gelegenen Stelle einen kleineren Durchmesser aufweist,

dadurch gekennzeichnet, daß der Ballon (7) in seinem zusammengezogenen Zustand über seine axiale Länge einen im wesentlichen gleichbleibenden Durchmesser aufweist,

so daß der Ballon (7) in einem aufgeblasenen Zustand axial über das distale Ende des langgestreckten Elements (2) hinausragt.

2. Ballon-Katheter nach Anspruch 1, ferner umfassend ein zweites Lumen (16), das axial durch das langgestreckte Element (2) zu dessen distalem Ende verläuft.

3. Ballon-Katheter nach Anspruch 1, wobei der Anschlußabschnitt sich unter einem Winkel von 0,85° bis 1,45° relativ zur Achse des langgestreckten Elements verjüngt.

4. Ballon-Katheter nach einem der vorangehenden Ansprüche, wobei der Vorderendabschnitt des Ballonmaterials am vorderen Bereich des Anschlußabschnitts (3) angebracht ist, der restliche Abschnitt des Ballonmaterials von innen nach außen gestülpt und über das langgestreckte Element in Richtung auf das hintere Ende des Anschlußabschnitts gezogen ist, und das Ballonmaterial gegen das hintere Ende des Anschlußabschnitts (3) angelegt und daran befestigt ist.

## Revendications

1. Cathéter à extrémité gonflable comprenant :

un élément (2) globalement cylindrique de forme allongée, comportant un axe géométrique, une extrémité distale et une partie de fixation (3) adjacente à l'extrémité distale et un ballonnet (7) dilatable/ contractable fixé à la partie de fixation dudit élément de forme allongée, et

un passage ou lumière (6) s'étendant à travers l'élément (2) de forme allongée et communiquant avec l'intérieur du ballonnet (7), la partie de fixation (3) ayant une forme conique qui converge vers l'extrémité distale et dont le diamètre plus petit se trouve à un endroit voisin de l'extrémité distale, **caractérisé** en ce que le ballonnet (7) a un diamètre sensiblement égal sur toute sa longueur axiale quand il se trouve dans l'état contracté, grâce à quoi le ballonnet (7) fait saillie axialement au-delà de l'extrémité distale de l'élément (2) de forme allongée quand il se trouve dans l'état dilaté.

2. Cathéter à extrémité gonflable selon la revendication 1, comprenant en outre un second passage ou lumière (16) s'étendant axialement à travers l'élément (2) de forme allongée jusqu'à l'extrémité distale.

3. Cathéter à extrémité gonflable selon la revendication 1, dans lequel la partie de fixation a une forme conique faisant un angle de 0,85° à 1,45° avec l'axe géométrique de l'élément de forme allongée.

4. Cathéter à extrémité gonflable selon l'une quelconque des revendications précédentes, dans lequel la partie d'extrémité avant du ballonnet est fixée à la zone avant de la partie de fixation (3), la partie restante du ballonnet étant rabattue vers l'intérieur et s'étendant sur l'élément de forme allongée en direction de l'extrémité arrière de la partie de fixation, et le ballonnet est appliqué contre l'extrémité arrière de la partie de fixation (3) et est fixé à cette extrémité arrière.

# F I G. 1

# F I G. 2

F I G. 3

# F I G. 4 PRIOR ART

## F I G. 5 PRIOR ART